# EUROPEAN PATENT APPLICATION

(11) **EP 4 434 482 A1**
(43) Date of publication of application: **25.09.2024**
(21) Application number: 23315057.2
(22) Date of filing: 21.03.2023
(51) Int. Cl.: A61B 34/00, A61B 34/30, A61B 1/00, A61B 17/00, A61B 1/005

(54) **A SYSTEM AND A ROBOTIC ENDOLUMINAL DEVICE FOR ADVANCEMENT IN A BODY DUCT OF A PATIENT**

(71) Applicant: Caranx Medical, 75013 Paris (FR)
(72) Inventor: BERTHET-RAYNE, Pierre, 06800 Cagnes-sur-Mer (FR); BENOIST, Camille, 06570 Saint-Paul-de-Vence (FR); SMITS, Jonas Victor Hamen, 3360 Bierbeek (BE)
(74) Representative: Hepp Wenger Ryffel AG

(57) **Abstract**

The invention relates to a system (101) for advancement in a body duct of a patient comprising a robotic endoluminal device (1) which has at least two nested concentric tubular elements (2, 3, 4) which are movable with respect to each other in a longitudinal direction (L) of the robotic endoluminal device (1). Bending sections (21, 31, 41) extend at least partially along the at least two concentric tubular elements (2, 3, 4) in the longitudinal direction (L). The robotic endoluminal device (1) has at least one deflecting means (5) per bending section (21, 31, 41) for deflecting the respective bending section (21, 31, 41) along a curve (C). The deflecting means (5) extend from a proximal end (102) of the system (101) to the bending section (21, 31, 41) .

## Description

The invention relates to a system and a robotic endoluminal device for advancement in a body duct of a patient according to the independent claims.

Gastrointestinal, endovascular or laparoscopic surgical procedures often require the advancement of an elongated device along a specific trajectory of a body duct, such as for placement of a prosthetic implant, e.g. of a heart valve prosthesis, placing a gastrointestinal implant or carrying out a gastrointestinal intervention, e.g. by entering through the mouth and oesophagus into the stomach, duodenum, jejunum or ileum.

In particular, endovascular procedures are associated with significant health risks due to plaque detachment, especially calcific detachment, caused by contact of the catheter with the walls of a bodily duct, in particular the ascending aorta, aortic arch, and abdominal aorta, which can lead to organ/vascular ischemia, e.g. by entering the carotid arteries, and may even be fatal.

Various catheters and guidewires known in the prior art are dependent on the skills of a clinician, in particular due to manual operation, are complex to control and/or manufacture, or not adapted to reliably avoid contact with the walls in a tortuous environment, e.g. the arch of the aorta. In particular, when performing retrograde or even antegrade transcatheter aortic valve implantations/replacements (TAVI/TAVR) reliable and precise advancement of a catheter and/or a guidewire to a valve annulus.

US 2016/346513 A1 discloses a small diameter bendable tip which allows a user to control a tip with an arc length varying curvature, e.g. by rotation and translation of an inner and outer tube with circumferentially asymmetric notches.

WO 2021/216782 A1 discloses a coaxially aligned steerable guidewire for a vascular system which provides a variable curvature and arc length of a distal end independently from each other.

However, there is generally a lack of a simple and reliable system/endoluminal device for advancement in body ducts which is deflectable to a tortuous course of a body duct. In particular, there is a lack of a transluminal device which enables a so called "follow the lead" steerability as described with a single degree of freedom at length in US 2016/346513 A1 in a lateral plane of the transluminal device when advancing/retracting the endoluminal device.

Moreover, the prior art fails to provide a robotic transluminal device deflectable to multi-directional curves and which allows for adjusting to different curves at different regions of the tortuous trajectory of the body duct.

It is an object of the present invention to overcome these and other disadvantages of the prior art. In particular, the invention shall provide a simple system and a robotic endoluminal device for advancement of a robotic endoluminal device within complex body ducts with a tortuous trajectory. Furthermore, the present invention shall provide improved handling of the robotic endoluminal device, preferably by fully automatic and/or remote control of the robotic endoluminal device.

These objects are solved by a system and a robotic endoluminal device for advancement in a body duct of a patient defined in the independent claims. Further embodiments are claimed in the dependent claims.

A system according to the invention is used for advancement in a body duct of a patient, in particular advancement from the femoral artery to the ascending aorta of a patient or advancement within a gastrointestinal tract of a patient. It comprises a robotic endoluminal device. The robotic endoluminal device has an outer concentric tubular element and at least one inner concentric tubular element which are nested within each other. An outer bending section extends at least partially along the outer concentric tubular element and at least one inner bending section extends at least partially along the at least one inner concentric tubular element in a longitudinal direction of the robotic endoluminal device. The robotic endoluminal device has at least one deflecting means per bending section for deflecting the respective bending section along a curve and freezing the bending section. The deflecting means extend from a proximal end of the system to the bending section. The system has at least one actuation unit for moving the concentric tubular elements in the longitudinal direction to longitudinal positions independently from each other.

A controller is part of the system or is operatively connected or connectable to the robotic endoluminal device.

The controller and the robotic endoluminal device are configured for sequentially: (i) moving the outer concentric tubular element to a first longitudinal position and deflecting the outer bending section to a first curve, (ii) keeping the shape of the outer bending section unchanged or at least increasing resistance to a deflection of the bending section and thereby limiting mobility (hereinafter referred to as "freezing"), by actuating the respective deflecting means, (iii) moving the at least one inner concentric tubular element to a second longitudinal position such that the inner bending section protrudes partially or completely from the outer bending section, (iv) deflecting the at least one inner bending section to a second curve, and (v) freezing the at least one inner bending section by the respective deflecting means.

The curve of the bending section in the context of the present application is defined by a deviation from a straight shape of the bending section, in particular a deviation defining at least one curvature in a region of the bending section, in particular a plurality of different curvatures in different regions of the bending section.

The respective bending section(s) may be adapted to be deflectable along a curve in a plurality of trajectories radially away from the longitudinal direction, in particular in any direction radially away from the longitudinal direction.

Within the present application, the proximal and distal directions are defined as the proximal direction toward an operator/clinician of the system/robotic endoluminal device and the distal direction away from the operator/clinician.

For the purposes of this patent application, freezing of the bending section is to be understood as a reduction in the deformability of the bending section so that the bending section deforms less or not at all when force is applied, particularly when force is applied laterally to the bending section, e.g. by other sections, in particular other bending sections. The freezing of the bending section can therefore suppress or reduce deflection of a longitudinal part of another bending section which is circumferentially enclosed by the frozen bending section. It may also be conceivable that a frozen bending section suppresses or reduces deflection of a longitudinal part of another bending section which circumferentially surrounds the frozen bending section.

The curves of different bending sections may be arrangeable in different spatial trajectories from each other.

The concentric tubular elements can have different radial dimensions, such that they can be nested within each other. The concentric tubular elements may be telescopically movably arranged with respect to each other.

The concentric tubular elements may be non-rotatably arranged with respect to each other. This allows maintaining a fixed spatial orientation of the concentric tubular elements to each other and thus allows for more precise alignment of the curves of the concentric tubular elements with respect to each other. Furthermore, non-rotatably arrangement of the concentric tubular elements further reinforces the structural integrity of the robotic endoluminal device.

The bending section(s) may comprise or consist of a metal, in particular nitinol or stainless steel, and/or an elastic material, such as plastic, in particular an elastomer. This allows for enhanced structural integrity of the bending section(s) and/or an improved lateral deflectability.

The concentric tubular element(s), in particular the bending section(s), may have fluid-tight walls, e.g. by being coated or partially formed by a plastic. The system may further have annular sealing elements which are arranged between adjacent concentric tubular elements, such that fluid-tightness of the robotic endoluminal device can be achieved, even when moving concentric tubular elements with respect to each other.

The bending section(s) may extend along a longitudinal length of 0.1 cm to 30 cm, in particular 1 cm to 20 cm, preferably 3 cm to 15 cm, of the concentric tubular elements. These dimensions allow for an optimized handling and operability at customary surgical conditions, e.g. for advancement within the aorta to the valve annulus of the patient.

The concentric tubular element(s) may be coated a hydrophilic coating to allow for reduced friction and thus enhanced advancement/compatibility with bodily fluids of the body duct, e.g. due to blood flow.

The concentric tubular element(s) and in particular the bending section(s) may have a reinforcement to protect against kinking or damaging of the concentric tubular element(s).

The bending sections may incrementally increase in deformability from the outer concentric tubular element to the innermost concentric tubular element, even when converted to a frozen configuration by actuation of the deflecting means. This allows e.g. that a part of an inner bending section which protrudes from an outer bending section/concentric tubular element can assume a curvature without being restricted by the outer concentric tubular element. However, the part of the inner bending section which is circumferentially surrounded by the outer bending section/concentric tubular element instead, can at least to a certain degree, assume the curvature of the outer bending section/concentric tubular element due to the higher deformability of the inner bending section, even when converted to the frozen configuration. This allows the length of the protruding part to be adapted to a particular bend of a body duct while the circumferentially surrounded part is not deflected or only deflected to a lower degree. The circumferentially surrounded part may therefore have a shape defined/at least partially defined by the outer tubular element, in particular the outer bending section.

The control unit can be configured for readjusting the first curve of the outer bending section based on the deflection/anticipated deflection of the inner bending section to a second curve.

The robotic endoluminal device can have at least a first and a second inner concentric tubular element. A second inner bending section extends along the second inner concentric tubular element in the longitudinal direction. The second inner concentric tubular element is nested within the first inner concentric tubular element. The controller is configured for sequentially moving the second inner concentric tubular element to at least a third longitudinal position such that the inner bending section protrudes partially or completely from the first inner bending section. The controller is further configured for deflecting the second inner bending section to a third curve and freezing the second inner bending section by the respective deflecting means.

This allows the robotic endoluminal device that the curve of the bending sections of each of the three concentric tubular elements can be controlled to be deflected, e.g. corresponding to a complex tortuous body duct. Furthermore, freezing the bending sections of the concentric tubular elements allows for adjusting the length of the curves of the bending sections, e.g. to the length of a bend/a plurality of bends of the tortuous body duct.

The controller may be adapted for modelling the mechanical strain the bending sections have on each other based one the deflection and/or the position of the bending sections with respect to each other. The model may be modelled based on geometrical data, statistical data, and/or mathematical data. This model may include a closed feedback loop data of the deflection, movement, and/or freezing of the bending sections, respectively. In addition, the device may include a pressure or a deformation sensor, in particular pressure or deformation sensors longitudinally offset from each other, which are connected to the controller to optimize the model.

The controller and the robotic endoluminal device may be adapted to control the deflection, the movement, and/or the freezing of the respective bending section(s) based on the model of the mechanical strain.

This allows load peaks of the bending sections/concentric tubular elements to be avoided and generally reduces the risk of a defect.

The individual concentric tubular element may have an increased longitudinal length from outermost to innermost concentric tubular element. The inner concentric element(s) with a lower radial dimension than the outer concentric tubular element can be configured to be advanced farther in the longitudinal direction than the outer concentric tubular element(s). This allows to enter narrower or branching body ducts, an obstruction or a vascular constriction, e.g. a heart valve annulus, with a section of the robotic endoluminal device with a smaller radial dimension.

The three concentric tubular elements may each have exactly one bending section, respectively. However, a plurality of bending sections may be arranged along different longitudinal sections of one of the concentric tubular elements. This can allow an optimized adaptation to a specific surgical procedure that requires a specific curvature in a well-defined region or curvatures in a plurality of specific regions which are spaced apart from each other in a known manner.

The control unit can be configured for readjusting the second curve of the first inner bending section based one the deflection/anticipated deflection of the second inner bending section to a third curve.

The outer concentric tubular element can have a stiffening concentric sleeve-like tubular element which is arrangeable circumferentially around the outer bending section and independently movable in the longitudinal direction, in particular by the actuation unit. A part of the outer bending section enclosed by the stiffening concentric tubular member cannot be deflected to the first curve or only deflected to a lesser degree than a protruding part of the outer bending section protruding from the stiffening concentric tubular member.

This allows to define a starting point of the first curve by the longitudinal positioning of the stiffening concentric tubular element with respect to the first bending section, so that a length of the curve of the first bending section can be adjusted.

The system can have at least two first actuation units for moving the nested outer concentric tubular element and the at least one inner concentric tubular element. In particular, the system can have three actuation units for moving the outer, first inner, and second inner concentric tubular elements independently from each other.

Each actuation unit can be adapted for operating the deflecting means and/or the movement of the concentric tubular elements in the longitudinal direction independently from each other.

Alternatively, different actuation units may be used for operating the deflecting means and for movement of the concentric tubular elements.

The actuation unit adapted for moving the concentric tubular elements in the longitudinal direction independently from each other can be fixedly arranged on the base, in particular at a proximal end of the base.

The actuation unit(s) may comprise or be connected to at least one electric motor, in particular a direct current motor, optionally an electric step motor.

The deflecting means may e.g. be formed by pull wires, microfluidic channels and/or push rods. The deflecting means may be actuatable by a separate actuation unit other than the actuation unit(s) for independently moving the nested concentric tubular element(s) which is spaced apart from the other actuation unit.

The bending section(s) may comprise a plurality of deflecting means directed at different longitudinal subsections of the bending section such that the curve of the bending section(s) can have a different curvature in the corresponding subsections. This may e.g. be achieved by a first group pull wires leading from a proximal end of the device to the distal end of a first longitudinal sub-section of the bending section and a second group of pull wires leading from the proximal end to the distal end of a second longitudinal subsection of the bending section.

The actuation unit(s) may be operatively connected to the controller for controlling the curves and longitudinal positions.

The proximal end of the at least one concentric tubular element may be operatively connected to the at least one first actuation unit and can be at least partially surrounded, in particular concentrically surrounded, by a stabilizing member for preventing bending of the proximal end of the concentric tubular element.

The concentric tubular elements being operatively connected to the at least one first actuation unit or an actuation unit respectively allows for operating the deflecting means and/or the movement of the concentric tubular element.

This allows to prevent excessive bending, buckling or even damaging of the concentric tubular elements when advancing or retracting the concentric tubular elements via the actuation unit.

The stabilizing member may be extendable in the longitudinal direction of the robotic endoluminal device or in a direction inclined at an angle between 0° and 30° with respect to the longitudinal direction when moving the concentric tubular element in the longitudinal direction. In particular the stabilizing member can be formed by a stabilizing spring element or a plurality of nested cylindrical elements which are telescopically extendable.

The extendable stabilizing member allows to prevent bending/damage to the concentric tubular elements irrespective of a longitudinal position of the proximal end of the concentric tubular elements.

The base may comprise a first member which is longitudinally fixed with respect to the base and a second member, which is operatively connected to one of the concentric tubular members. The first member e.g. can be a lead screw and that second member can be connection piece attached to the tubular member and threadably engaging the lead screw. The thread of the lead screw may substantially extend over the entire length of the lead screw. Alternatively, an arrangement with a gear and a rack may be used.

Preferably, the system may have at least one actuation unit of a second type for actuating the deflecting means, in particular a plurality of deflecting means individually from each other, which is connected to at least one concentric tubular element, in particular a proximal end of the concentric tubular element. Such a second actuation unit can be slidably connected to the base, in particular to a first member, e.g. a lead screw, of the base, such that the second actuation unit is movable along the base, in particular by the first actuation unit, in the longitudinal direction of the robotic endoluminal device synchronized with the movement of the concentric tubular element in the longitudinal direction.

The second actuation unit may be associated with the abovementioned second member for engaging the base, in particular for engaging the first member (e.g. a threaded lead screw), such that the second actuation unit can be reliably positioned with respect to the base when being moved in the longitudinal direction, e.g. via the lead screw having a corresponding interengaging thread.

This allows a secure fixation of the second actuation unit and enables reliable movement of the concentric tubular elements relative to each other. Moreover, a longitudinally slidable second actuation unit with respect to the base allows for a longitudinally extended design of the robotic endoluminal device which does not require bending of the proximal end of the robotic endoluminal device. This reduces the risk of damaging or buckling of the robotic endoluminal device.

The second actuation unit and the concentric tubular elements being movable in the longitudinal direction by actuation via the first actuation unit in a synchronized manner, in particular in unison, enable a constant actuateability of the deflecting means without them having to be adjusted in length for correcting the longitudinal position of the concentric tubular elements.

The first actuation unit for longitudinal movement of the concentric tubular element and the second actuation unit for actuation of the deflecting means may also both be movable along the longitudinal direction. This may simplify the design by requiring less electric connections and housing components.

The base, in particular the guide and/or the first member, may comprise or consist of metal, in particular stainless steel.

The base may comprise a plurality of guides and first members, in particular at least two, preferably at least three guides and first members. Each second actuation unit may be attached to or engage with a different guide and first member.

The actuation unit(s), in particular the second actuation unit(s), may be connected to the guides of the base, e.g. for maintaining a rotational orientation with respect to the base.

The first member may be arranged parallel to each other and in particular at different angular positions with respect to a longitudinal axis of the base. The first member(s) may comprise a thread which extends at least partially in particular completely along the first member(s). The angular positions of the first member may be arranged at different circumferential positions analogous to the deflecting means. In particular, the angular positions of the plurality of first member may be arranged at an angle of 60°, 90°, 120°, or 180° relative to the longitudinal axis.

The first member(s) may be fixed to the base at least at one of, in particular both of, a proximal end and a distal end of the first member (s).

A range of motion of the actuation unit, in particular the second actuation unit, and/or concentric tubular elements along the longitudinal direction preferably corresponds at least to a distance along which the concentric tubular elements are to be advanced in the body duct, in particular from the femoral artery to an aortic valve annulus.

The actuation unit, in particular the second actuation unit, and/or concentric tubular elements may be movable in the longitudinal direction within a maximum range of 160 cm, in particular 140 cm, preferably 120 cm.

The actuation units, in particular the second actuation units, may be sequentially positioned/positionable in the longitudinal direction of the base with respect to each other to account for a maximum range of movement of the actuation units and/or concentric tubular elements with respect to each other.

The system may have at least two, in particular at least three deflecting means, per bending section. The deflecting means may be spaced apart from each other in a circumferential direction or around the concentric tubular element and may be formed by pull wires which are preferably arranged within a low friction channel extending within the concentric tubular elements.

The deflecting means may lead to the distal end of a bending section at different circumferential positions. This allows to deflect the bending section(s) to curves indifferent directions. In a preferred embodiment, at least three deflecting means are connected to one bending section, respectively, such that a superposition of actuations of the deflecting means allows to control the curve of the bending section(s) in two degrees of freedom.

The actuation unit may be configured for operating the deflecting means, in particular by actuating the pull wires, independently from each other.

The deflecting means may be attached to the bending section at a terminal end of the distal end of the bending section. This allows for deflecting the entire bending section over its entire length by actuating the pull wires.

Freezing of the bending section(s) by deflecting means formed by pull wires can be achieved by simultaneously actuating the plurality of pull wires, e.g. after the bending section(s) being deformed to a desired curve. When freezing the bending section(s), the pull wires are controlled and/or arranged such that the actuation forces balance each other out. Thus, a longitudinal tension of the pull wires to the same extent can freeze the bending section(s) in a desired curve, e.g. the first, the second or the third curve.

The concentric tubular element or at least the bending sections of the concentric tubular elements may be formed by two concentric tubular sub-elements that are nested and fixedly connected together.

The low friction channel for receiving the pull wire may be formed by a recess in at least one, in particular both, the outer concentric tubular sub-element and the inner concentric tubular sub-element of a bending section. This allows easier manufacture of the low friction channel and placement of the pull wires in the low friction channel.

The low friction channel may be coated with a polymeric plastic, in particular polytetrafluoroethylene, to reduce the friction for actuating the pull wires within the low friction channel.

Alternatively or additionally to pull wires, the system may have push-rods or a microfluidic system for deflecting the bending section along the curve. The push-rods can be configured for a bi-directional movement along the longitudinal direction for applying a compressive and tensile force to the bending section.

The at least one bending section, in particular all bending sections, can extend from an intermediate section between a proximal end and a distal end of the respective concentric tubular element to the distal end of the concentric tubular element.

This allows the bending sections of the robotic endoluminal device to be alignable with respect to each other to form longitudinally directly adjacent bendable sections which are adapted to assume different curves, e.g. to manoeuvre through a tortuous body duct with different bends. Furthermore, this allows the plurality of longitudinally neighbouring protruding parts of the bending sections to be contiguous to each other, such that a longitudinal length of a total bendable region formed by the protruding parts can be adjustable by moving the concentric tubular elements with respect to each other.

The innermost concentric tubular element can concentrically surround at least one lumen of the robotic endoluminal device for insertion of surgical instruments, in particular insertion of a guidewire, an endoscope, a catheter, such as a drug delivery catheter, an end effector, and/or an implant.

This allows the clinician to carry out a surgical intervention, e.g. once the robotic endoluminal device has been completely advanced to a target site within the body duct. Furthermore, the innermost concentric bending section, e.g. the first inner or second inner bending section, may be adapted to be deformable by the guidewire if the respective deflecting elements are not actuated, such that the innermost bending section can be deformed following the three-dimensional shape of the guidewire.

A maximum outer radial dimension of the concentric tubular element(s) may be essentially constant along the length of the concentric tubular element(s).

This allows a seamless sliding when moving the concentric tubular elements with respect to each other.

The maximum outer radial dimension of the concentric tubular element(s) may vary within a range of 10%. In addition, the concentric tubular element(s) may have sealing elements that still allow seamless sliding and render the system fluid-tight when the concentric tubular elements are moved relative to each other.

The bending section(s) may comprise a plurality of at least partially circumferentially extending notches which are at least partially spaced apart in the longitudinal direction.

These notches allow for sufficient flexibility of the bending section(s) to be deflected while maintaining the structural integrity of the bending section. Thus, for example, buckling, or even damage to the bending section can be avoided.

The notches may be laser cut into the bending section. The bending section(s) or even the entire concentric tubular element(s) may be monolithic.

At least two notches, in particular at least three notches, may extend in succession along the circumferential direction of the bending section(s) and may be separated from each other by a material linker of the bending section(s).

The notches of a bending section may extend along different lengths in the longitudinal direction to account for a greater curvature in specific regions when deformed to the curve.

The material linkers ensure that the bending section is robust despite a plurality of notches being arranged along the longitudinal direction and provide sufficient flexibility for deflection to the curve(s) of the bending section(s).

The notches can be arranged symmetrically on the bending section, in particular point-symmetrically, with respect to a cross-sectional centre of the bending section.

Circumferential positions of material linkers of longitudinally adjacent notches may be offset from each other, in particular offset from each other, such that a circumferential centre of the notches is aligned with a longitudinally adjacent material linker in the longitudinal direction.

This circumferential positioning of material linkers and notches allows for an optimized deflection, e.g. in two degrees of freedom, of the bending section without compromising the structural integrity. Furthermore, a manufacturing process of the bending sections is simplified by allowing an alternating arrangement of material linkers in a pre-defined pattern which allows for easier automated machining.

The material linkers may be arranged at an angle with respect to each other relative to a central axis of the bending section of 60°, 90°, 120°, or 180°.

In particular, an angle of 120° between the material linkers relative to each other allows a good compromise of optimized deformability of the bending section and at the same time ensuring the structural integrity of the bending section.

The number and/or the interdistance between notches per a specific length in the longitudinal direction of at least one of the bending sections can be different to the number and/or interdistance of notches per a specific length in the longitudinal direction of a neighbouring bending section of a different concentric tubular element. This allows longitudinal displacement of the concentric tubular elements with respect to each other and lateral deflection of the bending sections by the deflecting means without a risk of interlocking the bending sections.

The robotic endoluminal device may comprise a mounting section for mounting an implant, in particular a prosthetic heart valve implant, or a gastrointestinal end effector, in particular a suturing device.

An end effector, e.g. a suturing device, of the device may be adapted for treating obesity of patients by suturing sections of the intragastric tract, reduction of intragastric volume, placement of implants within the gastrointestinal tract, or an anastomosis of sections of the gastrointestinal tract.

This allows the system to be used to carry out surgical interventions, in particular cardiac surgical interventions, e.g. a transcatheter aortic valve implantations/replacement (TAVI/TAVR) .

According to another aspect of the invention, a robotic endoluminal device for advancement in a body duct of a patient, in particular for advancement from the femoral artery to the ascending aorta of a patient or within a gastrointestinal tract of a patient, comprises an outer concentric tubular element and at least one inner concentric tubular element which are nested within each other and are independently movable in a longitudinal direction of the robotic endoluminal device to longitudinal positions. An outer bending section extends at least partially along the outer concentric tubular element and at least one inner bending section extends at least partially along the at least one inner concentric tubular element in the longitudinal direction. The robotic endoluminal device has at least one deflecting means per bending section for deflecting the respective bending section to a curve and freezing the bending section. The deflecting means extend from a proximal end of the robotic endoluminal device to the bending section. The robotic endoluminal device is adapted to be operatively controlled by a controller for moving the outer concentric tubular element to a first longitudinal position and deflecting the outer bending section to a first curve. Then the controller is configured for freezing the outer bending section by the respective deflecting elements. Thereupon, the controller is configured for moving the at least one inner concentric tubular element to at least a second longitudinal position such that the inner bending section protrudes partially or completely from the outer bending section, and for deflecting the at least one inner bending section to a second curve. Subsequently, the controller is configured for freezing the at least one inner bending section by the respective deflecting means.

The robotic endoluminal device can have at least a first and a second inner concentric tubular element. A second inner bending section extends at least partially along the second inner tubular element in the longitudinal direction and the second inner concentric tubular element is nested within the first inner concentric tubular element. The robotic endoluminal device is adapted to be operatively controlled by a controller for moving the second inner concentric tubular element to at least a third longitudinal position such that the inner bending section protrudes partially or completely from the first inner bending section and for deflecting the second inner bending section to a third curve. Thereupon, the controller is configured to control the robotic endoluminal device to freeze the second inner bending section by the respective deflecting means.

The invention further relates to a method for operating a system for advancement in a body duct of a patient, in particular for advancement from the femoral artery to the ascending aorta of a patient or within a gastrointestinal tract of a patient, comprising a robotic endoluminal device.

The robotic endoluminal device has an outer concentric tubular element and at least one inner concentric tubular element which are nested within each other.

An outer bending section extends at least partially along the outer concentric tubular element and at least one inner bending section extends at least partially along the at least one inner concentric tubular element in a longitudinal direction of the robotic endoluminal device.

The method further comprising moving an outer concentric tubular element to a first longitudinal position and deflecting the outer bending section to a first curve.

The method includes freezing the outer bending section by the respective deflecting means, moving the at least one inner concentric tubular element to at least a second longitudinal position such that the inner bending section protrudes partially or completely from the outer bending section and deflecting the at least one inner bending section to a second curve.

The method further comprises freezing the at least one inner bending section by the respective deflecting means.

The invention is now described with reference to certain embodiments and figures which show:
- Figure 1:: a perspective side view of a system having a robotic endoluminal device with three concentric tubular elements and bending sections;
- Figures 2A and 2B:: a side view of the second inner concentric tubular element of the robotic endoluminal device of Fig. 1 with a bending section in a straight configuration and a bending section in three deflected configurations;
- Figure 2C:: a cross-sectional view of the inner concentric tubular element according to Figs. 2A and 2B;
- Figures 3A and 3B:: a side view and a perspective front view of the robotic endoluminal device of Fig. 1;
- Figures 4A to 4C:: side views of the robotic endoluminal device with an outer and inner concentric tubular element with bending sections being deformed to a first and a second curve;
- Figures 4D to 4G:: side views of the robotic endoluminal device of Fig. 1 with an outer, a first inner, and a second inner concentric tubular element with bending sections being deformed to a first, a second, and a third curve respectively;
- Figure 4H:: a side view of the robotic endoluminal device of Fig. 1 with retracted first inner and second inner concentric tubular elements;
- Figures 5A and 5B:: perspective side views of a proximal end of a first embodiment of the system with a base and a controller and an enlarged perspective side view of the proximal end of the base;
- Figures 6A and 6B:: perspective side views of the proximal end of a second embodiment of the system with a base with an elongateable stabilizing member and a controller.

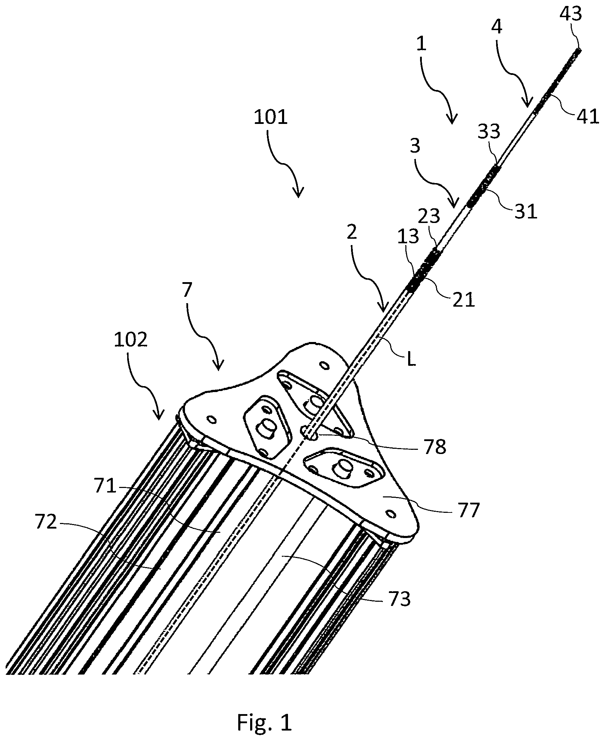

Figure 1 shows a perspective side view of a system 101 having a robotic endoluminal device 1 with three concentric tubular elements 2, 3, 4. The three concentric tubular elements 2, 3, 4 are nested within each other, such that a second inner concentric tubular element 4 extends from a first inner concentric tubular element 3 and the first inner concentric tubular element 3 extends from an outer concentric tubular element 2 as shown in Fig. 1. Each of the concentric tubular elements 2, 3, 4 has a bending section 21, 31, 41 extending partially in a longitudinal direction L of the robotic endoluminal device 1 up to a distal end 23, 33, 43 of the concentric tubular elements 2, 3, 4. An outer surface of the concentric tubular elements 2, 3, 4 is coated with a hydrophilic coating 13 for enhancing the compatibility with respect to bodily fluids, e.g. blood. In addition, the hydrophilic coating 13 improves a biochemical compatibility, such as enhancing non-thrombogenicity.

A base 7 of the system 101 is disposed at a proximal end 102 of the system 101. The base 7 has three lead screws 71, 72, 73 that are arranged parallel to the longitudinal direction L of the robotic endoluminal device 1. The lead screws 71, 72, 73 are fixedly attached to a distal base plate 77 of the base 7 at 120° with respect to a longitudinal axis along which the robotic endoluminal device 1 can be arranged centrally within the base 7. The lead screws 71, 72, 73 are configured to be operatively engaged by first actuation units 61, 62, 63 which are arranged at a proximal end of the base 7, having electric motors.

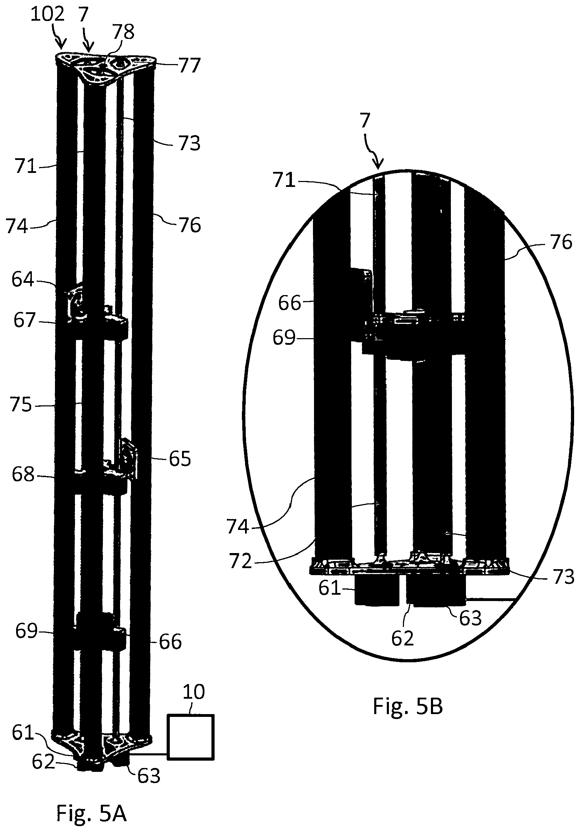

Each of the concentric tubular elements 2, 3, 4 is movable independently in the longitudinal direction L of the robotic endoluminal device 1 by a separate first actuation unit 61, 62, 63 (see Figs. 5A to 6B). Each of the first actuation units 61, 62, 63 is configured for rotating the lead screws 71, 72, 73. A thread of the lead screws 71, 72, 73 engages with a thread (not shown in Figs. 5A - 6B) of a housing of the second actuation unit 67, 68, 69, such that a rotation of the lead screws 71, 72, 73 allows for moving the second actuation unit 67, 68, 69 and the respective concentric tubular element 2, 3, 4 in the longitudinal direction.

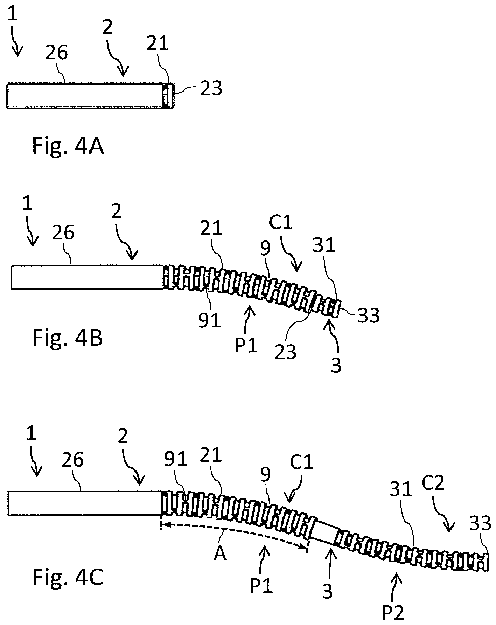

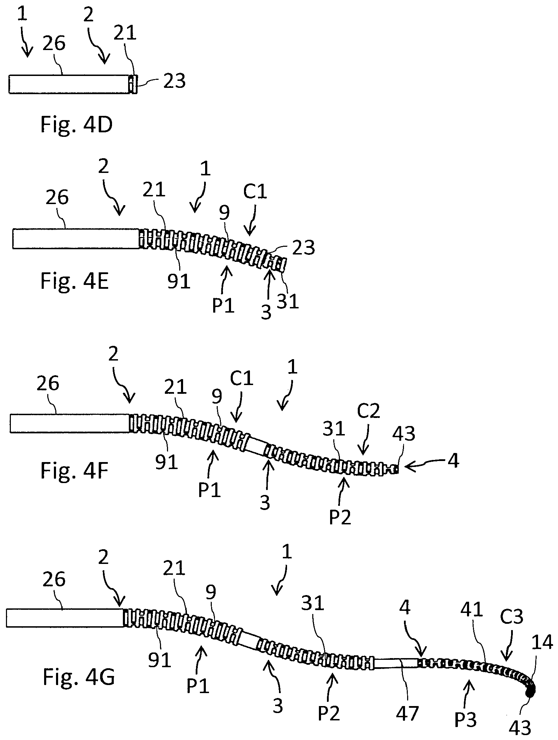

This allows to adjust a length of a protruding portion of second inner concentric tubular element 4 from the distal end 33 of the first inner concentric tubular element 3 and of a protruding portion from the first inner concentric tubular element 3 from the distal end 23 of the outer concentric tubular element 2 (see Figs. 4A - 4G).

Moreover, the protruding portion of the bending sections 21, 31, 41 can be deflected to a plurality of curves C1, C2, C3 (see Figs. 4A - 4G) along two degrees of freedom respectively by a controller controlling a plurality of deflecting means, formed by pull wires, via the second actuation units 67, 68, 69.

Each second actuation unit 67, 68, 69 is operatively connected to the three deflecting means of the concentric tubular elements 2, 3, 4 respectively and adapted to operate the deflecting means independently from each other.

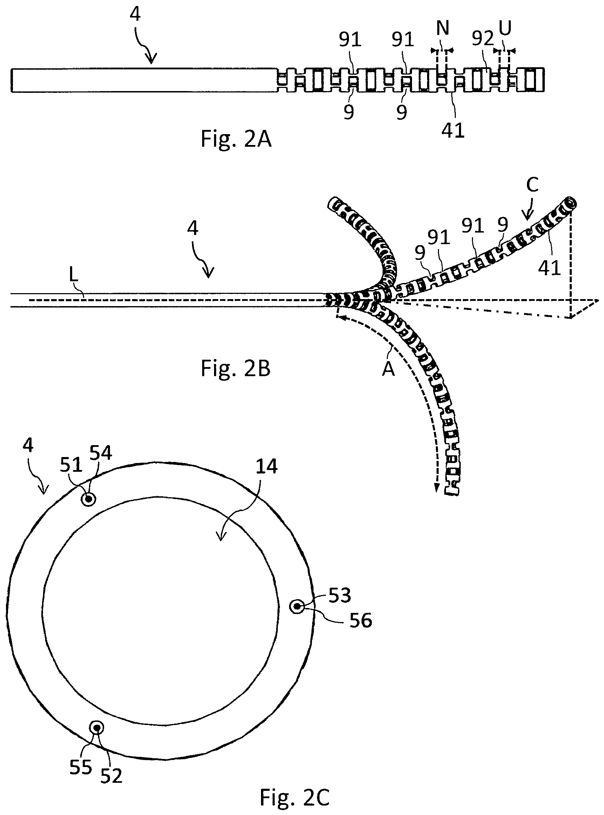

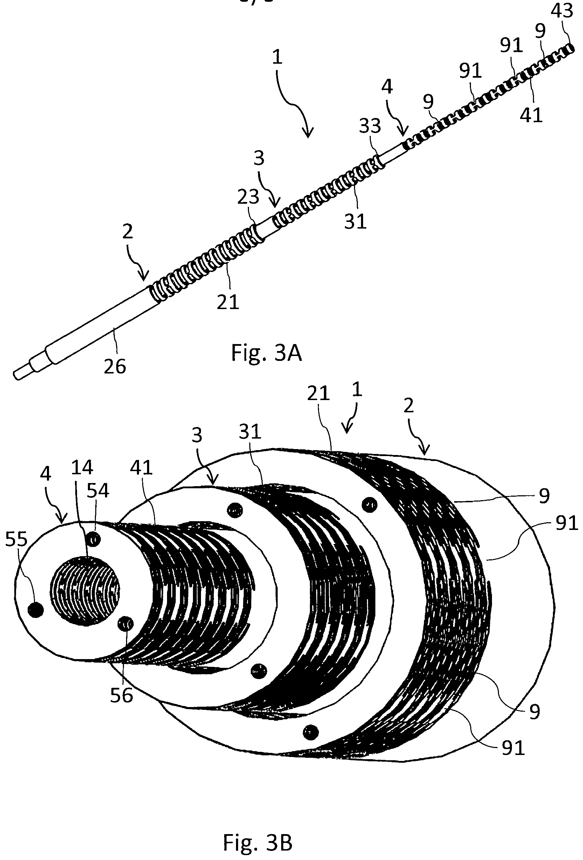

The inner concentric tubular element 4 circumferentially encloses a lumen 14 which may be e.g. used for insertion of surgical instruments when the robotic endoluminal device 1 was advanced to a target site within a body duct of a patient (see e.g. Figs. 2C and 3B).

Figures 2A and 2B show a side view of the second inner concentric tubular element 4 with the second inner bending section 41 of Fig. 1 in a straight configuration (Fig. 2A) and being deflected to three different curves C in a deflected configuration (Fig. 2B).

For better visibility of the bending section 41 Figs. 2A and 2B show an exposed stainless-steel core of the bending section 41. However, the bending section 41 may be coated or circumferentially enclosed by an elastomer such that it is fluid tight and essentially has a constant diameter along a longitudinal direction of the concentric tubular element 4.

The bending section 41 of the second inner concentric tubular element 4 has a plurality of notches 9. Groups of three notches 9 are arranged circumferentially adjacent from each other and spaced apart by material linkers 91 of the bending section 41 in a circumferential direction. These groups of three notches 9 and material linkers 91 are alternatingly arranged in a longitudinal direction L of the concentric tubular element 4 with circumferentially closed annular sections 92 of the bending section 41. This allows sufficient flexibility of the bending section 41 paired with sufficient structural integrity of the bending section 41.

The longitudinally adjacent material linkers 91, which are spaced apart by an annular section 92, are arranged circumferentially at an angle of 120° to each other with respect to the longitudinal axis of the concentric tubular element 2. This allows that the material linkers 91 are essentially aligned on a common longitudinal axis extending through a circumferential center position of the notches 9. By varying the circumferential position of the notches 9/the material linkers 91 alternatingly along the longitudinal direction L, the flexibility of the bending section 41 can be optimized without compromising the structural integrity of the concentric tubular element 4. Fig. 2B shows that this design of the bending section 41 allows the bending sections 41 to be deflected in two degrees of freedom to a plurality of curves C with respect to the longitudinal direction L, e.g. by being controlled by pull wires 51, 52, 53 (see Fig. 2C). This is exemplary shown in Fig. 2B by the bending section 41 assuming three different curves C along the length A of the bending section 41. The longitudinal length A of the bending section 41 which can freely assume a curve C may however be limited, e.g. by a surrounding frozen bending section 41 or a stiffening concentric tubular element (see Figs. 4A - 4G).

A length N of the notches 9 in Figs. 2A and 2B substantially corresponds to a longitudinal length U of the annular sections 92. However, the length N of the notches 9 and the length U of the annular sections 92 may differ from each other up to a factor of up to two.

Figure 2C shows a cross-sectional view of the second inner concentric tubular element 4 according to Figs. 2A and 2B. The second inner concentric tubular element 4 encloses three low friction channels 54, 55, 56 which extend along the longitudinal direction L of the concentric tubular element 4.

Within the three low friction channels 54, 55, 56, a pull wire 51, 52, 53 is arranged respectively, which can be actuated by the second actuation unit 67, 68, 69 in the longitudinal direction to bend the bending section 41 along a curve (see Fig. 2B, C and Figs. 5A to 6B). The pull wires 51, 52, 53 are attached to a distal end of the bending section 41 at three different circumferential positions which are spaced apart by 120° with respect to a center of the bending section 41. This allows for a deflection of the bending section 41 in two degrees of freedom.

The channels 54, 55, 56 are coated/formed by a material, e.g. poly(tetra)fluoroethylene, which provides a low frictional resistance with regard to a longitudinal movement of the pull wires 51, 52, 53.

The outer and first inner concentric tubular elements 2, 3 and outer and first inner bending sections 21, 31 of Fig. 1 are essentially shaped and formed analogous in structure to the second inner concentric tubular element 4 and second inner bending section 41 as previously described with regard to Figs. 2A and 2B.

The inner concentric tubular element 4 circumferentially encloses a lumen 14 along the longitudinal direction L which is configured for receiving a guidewire.

Figures 3A and 3B show a side view and a perspective front view of the robotic endoluminal device 1 of Fig. 1. The notches 9 and material linkers 91 of the concentric tubular elements 2, 3, 4 in Figs. 3A and 3B are arranged as previously described with regard to Figs. 2A and 2B. The outer, first inner, and second inner concentric tubular elements 2, 3, 4 with the respective bending sections 21, 31, 41 can be retracted and advanced with respect to each other in a longitudinal direction to adjust the positions of the bending sections 21, 31, 41 and/or a length A of a protruding portion of the bending sections 21, 31 with respect to a distal end 23, 33 of a surrounding concentric tubular element 2, 3 or a stiffening concentric tubular element 26. This telescopic movement of the concentric tubular elements 2, 3, 4 allows to align the bending sections 21, 31, 41 with a tortuous trajectory of a body duct and adjust the bending sections 21, 31, 41 individually from each other to correspond tortuous trajectory without contacting the wall.

Fig. 3B shows that each of the bending sections 21, 31, 41 has three channels 54, 55, 56 formed within the concentric tubular elements 2, 3, 4 for receiving a pull wire such that each bending section 21, 31, 41 may be deflected by pull wires 51, 52, 53 to a desired curve (see Fig. 2C).

Figures 4A to 4C show side views of a second embodiment of the robotic endoluminal device 1 which has an outer concentric tubular element 2 and an inner tubular element 3 with bending sections 21, 31 being deformed to a first and second curve C1, C2. The bending sections 21, 31 extend along the concentric tubular elements 2, 3 to the respective distal ends 23, 33 of the concentric tubular elements 2, 3.

Figure 4A shows the outer concentric tubular element 2 with the outer bending section 21 being circumferentially enclosed by a stiffening concentric tubular element 26 such that only the distal end 23 of the outer concentric tubular element 2 protrudes from the stiffening concentric tubular element 26. The outer bending sections 21, 31 have a plurality of notches 9 and material linkers 91 which are arranged as previously described in Figs. 2A and 2B.

Figure 4B shows the outer concentric tubular element 2 being moved in a longitudinal direction of the robotic endoluminal device 1 with respect to the stiffening concentric tubular element 26 to a first longitudinal position P1. Fig. 4B shows that protruding part of the outer bending section 21 with respect to the stiffening concentric tubular element 26 was deflected to a first curve C1 by actuating a deflecting means similar to Figs. 2A to 2C. Subsequently, the protruding part of the bending section 21 with a length A was converted to a frozen configuration while being deflected to the first curve C1 by simultaneously actuating the deflecting means. This can for example be achieved by actuating the pull wires 51, 52, 53 (see Fig. 2C) to deflect the protruding part of the outer bending section 21 to the first curve C1 and subsequently simultaneously actuating all pull wires 51, 52, 53 to convert the protruding part of the outer bending section 21 to the frozen configuration.

At least the distal tip of the stiffening concentric tubular element 26 has a greater stiffness than the outer concentric tubular element 2 in the frozen configuration, such that the distal tip of the stiffening concentric tubular element 26 is not deflected or only deflected to a lower degree with respect to the first curve C1.

The bending sections 21, 31 in Figs. 4A to 4C may be movable in the longitudinal direction with respect to each other either in a flexible configuration or in a deflected configuration with a first or second curve C1, C2.

Figure 4C shows the inner concentric tubular element 3 being moved in the longitudinal direction of the robotic endoluminal device 1 with respect to the outer concentric tubular element 2 to a second longitudinal position P2, such that the inner bending section 31 completely protrudes from the distal end 23 of the outer concentric tubular element 2.

Fig. 4C shows that the inner bending section 31 was deflected to a second curve C2 by actuating the respective deflecting means. Subsequently, the inner bending section 31 in Fig. 4C was converted to a frozen configuration while being deflected to the second curve C2 by simultaneously actuating the deflecting means. This can for example be achieved by actuating the pull wires 51, 52, 53 (see Fig. 2C) to deflect the completely protruding inner bending section 31 to the second curve C2 and subsequently simultaneously actuating all pull wires 51, 52, 53 to convert the inner bending section 31 to the frozen configuration. However, the inner bending section 31 in Fig. 4C may also only partially protrude from the outer concentric tubular element 2, such that only the protruding portion of the inner bending section 31 is deflected to the second curve C2 while the circumferentially surrounded section of the inner bending section 31 is at least partially confined by the frozen outer bending section 21. The inner bending section 31 which is circumferentially surrounded thus at least partially or completely assumes the shape of the frozen outer bending section 21.

Figures 4D to 4G show side views of the robotic endoluminal device 1 of Fig. 1 with an outer, a first inner, and a second inner concentric tubular element 2, 3, 4 with respective bending sections 21, 31, 41 being sequentially deformed to a first, a second, and a third curve C1, C2, C3 at a first, a second, and a third longitudinal position P1, P2, P3. The bending sections 21, 31, 41 comprise previously described notches 9 and material linkers 91 (see e.g. Figs. 2A and 2B).

The different curves C1, C2, C3 may e.g. correspond to a tortuous body duct of a patient with multiple bends. As shown in Figs. 4A to 4C and Figs. 4D - 4G the bending section 21, 31, 41 can be respectively deflected to a different curve, such that the robotic endoluminal device 1 can be advanced along complex trajectories.

The process of moving of the outer and the first inner concentric tubular element 2, 3 to longitudinal positions P1, P2, deflecting the tubular elements 2, 3 to a first and second curve C1, C2, and freezing the protruding part of the outer bending section 21 with respect to a stiffening concentric tubular element 26 and the complete first inner bending section 31 of the first inner concentric tubular element 3 is essentially analogous as previously described in Figs. 4A to 4C.

However, as shown in Figs. 4F and 4G the robotic endoluminal device 1 has a second inner concentric tubular element 4 with a second inner bending section 41. Fig. 4F shows that the second inner concentric tubular element 4 is circumferentially surrounded by the first inner concentric tubular element 3 which was deflected to a second curve C2 at a second longitudinal position P2 and subsequently converted to a frozen configuration.

Figure 4G shows that the second inner concentric tubular element 4 was moved in the longitudinal direction of the robotic endoluminal device 1 with respect to the first inner concentric tubular element 3 to a third longitudinal position P3, such that the second inner bending section 41 completely protrudes from a distal end 33 of the first concentric tubular element 3.

Fig. 4G shows that the second inner bending section 41 was deflected to a third curve C3 by actuating the respective deflecting means. Subsequently, the inner bending section 31 in Fig. 4G was converted to a frozen configuration while being deflected to the third curve C3 by simultaneously actuating the deflecting means. This can for example be achieved by actuating the pull wires 51, 52, 53 (see Fig. 2C) to deflect the completely protruding second inner bending section 41 to the third curve C3 and subsequently simultaneously actuating all pull wires 51, 52, 53 to convert the second inner bending section 41 to the frozen configuration.

The deflecting and freezing of the bending sections by actuation of the deflecting means and longitudinal movement of the concentric tubular elements 2, 3 by actuation units 61, 62, 63, 67, 68, 69 in Figs. 4A to 4G can be controlled via a controller (see e.g. Figs. 5A to 6B), in particular in a fully automated manner.

The second inner bending section 41 in Fig. 4G may also only partially protrude from the first inner concentric tubular element 3, such that only the protruding portion of the second inner bending section 41 is deflected to the third curve C3 while the circumferentially surrounded section of the second inner bending section 41 is at least partially confined by the frozen first inner bending section 31. The confined section of the second inner bending section 41 thus partially or completely assumes the shape of the surrounding frozen first inner bending section 31.

Figure 4G shows that the second inner concentric tubular element 4 has a mounting section 47 for receiving a prosthetic heart valve implant, such that a retrograde transfemoral TAVI/TAVR surgical intervention can be carried out using the robotic endoluminal device 1 in a manner known to the skilled person.

Alternatively, the mounting section 47 may be adapted for receiving a gastrointestinal end effector such as a suturing device. The mounting section 47 for receiving the end effector may be arranged at the distal tip of the concentric tubular element 4 or the end effector may be fixedly attached to the distal tip of the concentric tubular element 4. The device may be adapted for being advanced through the mouth and oesophagus into the stomach or intestine, in particular duodenum, jejunum, or ileum.

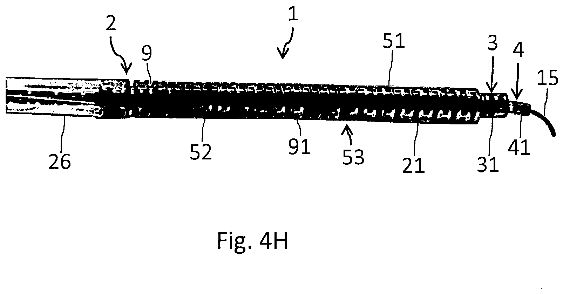

A lumen 14 of the second inner concentric tubular element 4 extends up to a distal end 43 and allows for introducing surgical instruments, e.g. a guidewire (see Fig. 4H).

Each bending section 21, 31, 41 or protruding part of the bending section 21, 31, 41 can be deflected to a different curve in two degrees of freedom, such that the robotic endoluminal device 1 can be advanced along complex trajectories.

Figure 4H shows a side view of the robotic endoluminal device 1 of Fig. 1 with the first inner and the second inner concentric tubular elements 3, 4 with respective bending sections 31, 41 retracted into the outer concentric tubular elements 2. The outer concentric tubular element 2 has a bending section 21 which assumes a straight configuration due to the deflecting means formed by three pull wires 51, 52, 53 which are arranged at 120° to a longitudinal axis of the robotic endoluminal device 1 not being actuated. Fig. 4H shows that a surgical instrument in the form of a guidewire 15 was advanced through a lumen 14 of the second inner concentric tubular element 4 (see Fig. 2C) to the distal opening of the concentric tubular element 4. The dimensions of the lumen 14 are sufficient, such that the guidewire 15 is rotatably and longitudinally displaceable within the lumen 14.

The robotic endoluminal device 1 may be used to be retracted after successfully placing the guidewire 15 from the lumen 14 for carrying out a surgical procedure, e.g. a TAVI/TAVR procedure. Alternatively, the robotic endoluminal device 1 may be used itself for carrying out the surgical procedure without requiring a lumen 14 with a guidewire 15, e.g. by having a mounting section 47 for a heart valve prosthesis for carrying out TAVI/TAVR on the robotic endoluminal device 1.

Figures 5A and 5B show a perspective side view of a proximal end 102 of the system with the base 7 and a controller 10. Fig. 5B shows an enlarged perspective side view of the proximal end of the base 7. Three first actuation units 61, 62, 63 having three electric motors are attached to three parallel lead screws 71, 72, 73 at a proximal end of the base 7. The three second actuation units 67, 68, 69 for actuating the deflecting means are further attached/attachable to proximal ends of three respective concentric tubular elements of a robotic endoluminal device 1 (see Figs. 1 - 4H) which are mounted through an opening 78 in the distal base plate 77 (see e.g. Fig. 1).

The parallel lead screws 71, 72, 73 are respectively also connected via connecting plates 64, 65, 66 to guide rails 74, 75, 76 which are placed radially outwardly with respect to the lead screws 71, 72, 73 with respect to the cross-sectional centre of the base 7. The connecting plates 64, 65, 66 are formed to engage the guide rails 74, 75, 76, such that each of the second actuation units 67, 68, 69 are movable by actuation of the first actuation units 61, 62, 63 along the lead screws 71, 72, 73 in the longitudinal direction between a proximal base plate 79 and the distal base plate 77.

A longitudinal length of the base 7 corresponds at least to the length the robotic endoluminal device can be advanced, in particular a length between 20 cm to 180 cm, such that the robotic endoluminal device does not need to be bent/wound up prior to insertion/advancement into the body duct.

The second actuation units 67, 68, 69 comprising separate electric motors are spaced apart from each other in a longitudinal direction of the base 7 such that they can move the concentric tubular elements 2, 3, 4 (see e.g. Fig. 1) in the longitudinal direction.

The second actuation units 67, 68, 69 are further configured to actuate the groups of three pull wires 51, 52, 53 (see Fig. 2C) which are connected to the respective bending sections of the concentric tubular elements. Each individual pull wire 51, 52, 53 of the pairs of three pull wires 51, 52, 53 may be actuated independently, e.g. to not adjusting the curve of the bending section and/or all together for freezing the bending section.

The longitudinal movement of the concentric tubular elements by the first actuation unit 61, 62, 63 and the actuation of the pull wires 51, 52, 53 by the second actuation unit 67, 68, 69 can be controlled independently from each other via the controller 10 which is operatively connected to the electric motors of the first actuation units 61, 62, 63 and the second actuation units 67, 68, 69 respectively. This allows to control the longitudinal movement of the three concentric tubular elements by the controller 10, independently from each other by rotating the lead screws 71, 72, 73 by actuation of the first actuation units 61, 62, 63 (see Figs. 5A and 5B). The lead screws 71, 72, 73 have a threaded section which is configured to engage a threaded section of the housing of the second actutation units 67, 68, 69 respectively. The threaded sections of the housing allows for converting the rotational movement to a movement in the longitudinal direction L along the lead screws 71, 72, 73 and guide rails 74, 75, 76. The guide rails 74, 75, 76 prevent a rotation of the second actuation units 67, 68, 69 during the rotational movement of the lead screw 71, 72, 73 due to being connected to the connecting plates 64, 65, 66.

The controller 10 is further configurated to perform a follow-the-lead longitudinal advancement and corresponding deflection of the robotic endoluminal device by controlling the first and second actuation units 61, 62, 63, 67, 68, and 69 in a coordinated manner. This allows the robotic endoluminal device 1 to be advanced along a specific trajectory, e.g. within a body duct, with minimal deviations such that the risk of contacting the walls of the body duct is minimized.

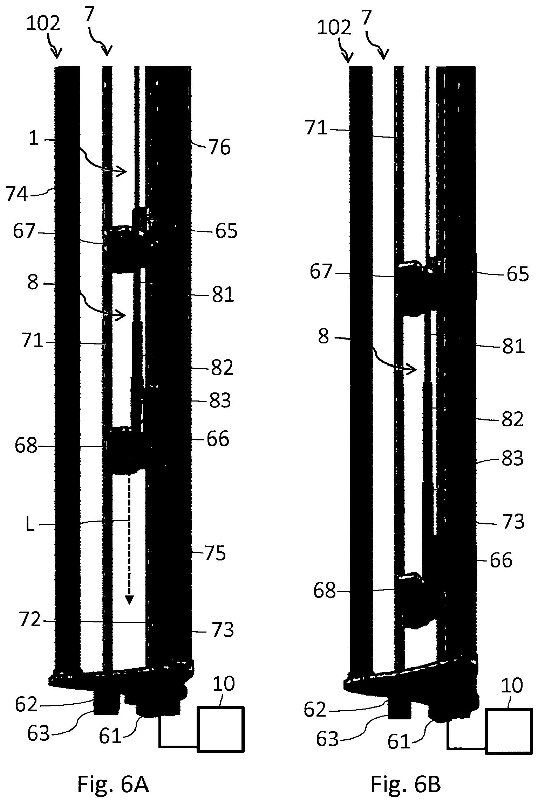

Figures 6A and 6B show a perspective side view of a proximal end 102 of a second embodiment of the system with a base 7 with an extendable stabilizing member 8 and a controller 10.

The second embodiment of the system also comprises a previously described base 7 with lead screws 71, 72, 73, guide rails 74, 75, 76, and first and second actuation units 61, 62, 63, 67, 68, 69 (see Figs. 5A and 5B). Figs. 6A and 6B show that the concentric tubular elements of the robotic endoluminal device 1 are mounted to the second actuation units 67, 68, 69. In a first position shown in Fig. 6A, the section of the second inner concentric tubular element 4 (see Fig. 1) of the robotic endoluminal device 1 is arranged partially between the two second actutation units 68, 69 and circumferentially enclosed by the stabilizing member 8 formed by three nested steel cylinders 81, 82, 83. The second inner concentric tubular element 4 is operatively connected for a longitudinal movement to the proximalmost second actuation unit 69.

Fig. 6B shows a second position in which the proximalmost second actuation unit 69 was moved by the first actuation unit 63 along a longitudinal direction L of the base 7 for retracting a concentric tubular element of the robotic endoluminal device 1. The nested steel cylinders 81, 82, 83 in Fig. 6B are telescopically extended in the longitudinal direction L, such that the robotic endoluminal device 1 can be protected from bending/breaking even when exerting force by movement of the second actuation unit 69 connected to the second inner concentric tubular element.

The outer concentric tubular elements 2 (see Fig. 1) of the robotic endoluminal device 1 is instead attached to the distalmost second actuation unit 67 and the first inner concentric tubular element 3 is attached to the intermediated second actuation unit 68. This allows for a reliable and secure engagement of the second actuation units 67, 68, 69 to the concentric tubular elements 2, 3, 4 for longitudinal movement of the concentric tubular elements 2, 3, 4 and deflection of the corresponding bending section 21, 31, 41.

The first and second actuation units 61, 62, 63, 67, 68, 69 may each comprise a motor for rotating the lead screws and for moving the pull wire, e.g. wrapped on a pulley. The motors may be motors conventionally used in robotics, e.g. supplied by Dynamixel.

## Claims

1. A system (101) for advancement in a body duct of a patient, in particular for advancement from the femoral artery to the ascending aorta of a patient or advancement within a gastrointestinal tract of a patient, comprising a robotic endoluminal device (1) which has
an outer concentric tubular element (2) and at least one inner concentric tubular element (3, 4) which are nested within each other, wherein
an outer bending section (21) extends at least partially along the outer concentric tubular element (2) and at least one inner bending section (31, 41) extends at least partially along the at least one inner concentric tubular element (3, 4) in a longitudinal direction (L) of the robotic endoluminal device (1), and
at least one deflecting means (51, 52, 53) per bending section (21, 31, 41) for deflecting the respective bending section (21, 31, 41) along a curve (C1, C2, C3) and freezing the bending section (21, 31, 41), the deflecting means (51, 52, 53) extending from a proximal end (102) of the system (101) to the bending section (21, 31, 41), wherein the system (101) has at least one actuation unit (61, 62, 63, 67, 68, 69) for moving the concentric tubular elements (2, 3, 4) in the longitudinal direction (L) to longitudinal positions (P1, P2, P3) independently from each other, wherein
a controller (10) is part of the system (101) or operatively connected or connectable to the system (101), **characterized in that** the controller (10) and the robotic endoluminal device (1) are configured for sequentially:
- Moving the outer concentric tubular element (2) to a first longitudinal position (P1) and deflecting the outer bending section (21) to a first curve (C1),
- Freezing the outer bending section (21) by the respective deflecting means (51, 52, 53),
- Moving the at least one inner concentric tubular element (3, 4) to at least a second longitudinal position (P2, P3) such that the inner bending section (31, 41) protrudes partially or completely from the outer bending section (21) and deflecting the at least one inner bending section (31, 41) to a second curve (C2, C3), and
- Freezing the at least one inner bending section (31, 41) by the respective deflecting means (51, 52, 53).

2. The system (101) according to claim 1, wherein the robotic endoluminal device (1) has at least a first inner concentric tubular element (3) and a second inner concentric tubular element (4), wherein a second inner bending section (41) extends at least partially along the second inner concentric tubular element (4) in the longitudinal direction (L) and
the second inner concentric tubular element (4) is nested within the first inner concentric tubular element (3), wherein the controller (10) is configured for:
- Moving the second inner concentric tubular element (4) to at least a third longitudinal position (P3) such that the inner bending section (41) protrudes partially or completely from the first inner bending section (31) and for deflecting the second inner bending section (41) to a third curve (C3), and
- Freezing the second inner bending section (41) by the respective deflecting means (51, 52, 53).

3. The system (101) according to one of the preceding claims, wherein the outer concentric tubular element (2) has a stiffening concentric sleeve-like tubular member (26) which is arrangeable circumferentially around the outer bending section (21) and independently movable in the longitudinal direction (L), in particular by the actuation unit (61, 62, 63) so that an enclosed part of the outer bending section (21) enclosed by the stiffening concentric tubular member (26) cannot be deflected to the first curve (C1) or deflected to the first curve (C1) to a lesser degree than a protruding part of the outer bending section (21) protruding from the stiffening concentric tubular member (26).

4. The system (101) according to one of the preceding claims, wherein the system (101) has at least two first actuation units (61, 62, 63) for moving the nested outer concentric tubular element (2) and the at least one inner concentric tubular element (3, 4), in particular having three actuation units (61, 62, 63) for moving the outer, first inner, and second inner concentric tubular elements (2, 3, 4) independently from each other.

5. The system (101) according to claim 4, wherein the proximal end (22, 32, 42) of at least one of the concentric tubular element (2, 3, 4) is operatively connected to the at least one first actuation unit (61, 62, 63, 67, 68, 69) and is at least partially surrounded, in particular concentrically surrounded, by a stabilizing member (8) for preventing bending of the proximal end (22, 32, 42) of the concentric tubular element (2, 3, 4).

6. The system (101) according to claim 5, wherein the stabilizing member (8) is configured to be extendable in the longitudinal direction (L) of the robotic endoluminal device (1) when moving the concentric tubular element (2, 3, 4) in the longitudinal direction (L) or a direction inclined at an angle between 0° and 30° with respect to the longitudinal direction (L), in particular by the stabilizing member (8) being formed by a stabilizing spring element or a plurality of nested cylindrical elements (81, 82, 83) which are telescopically extendable.

7. The system (101) according to one of the preceding claims, wherein the system (101) has at least one second actuation unit (67, 68, 69) for actuating the deflecting means (51, 52, 53) which is connected to at least one concentric tubular element (2, 3, 4), in particular to a proximal end of the concentric tubular element (2, 3, 4), and the second actuation unit (67, 68, 69) is preferably slidably connected to a base (7), in particular to a first member (71, 72, 73) of the base (7), such that the second actuation unit (67, 68, 69) is movable, in particular by the first actuation unit (61, 62, 63), in the longitudinal direction (L) of the robotic endoluminal device (1) synchronized with the movement of the concentric tubular element (2, 3, 4) in the longitudinal direction (L).

8. The system (101) according to one of the preceding claims, wherein the system (101) has at least two deflecting element (51, 52, 53), in particular at least three deflecting means (51, 52, 53), per bending section (21, 31, 41), wherein the deflecting means (51, 52, 53) are spaced apart from each other in a circumferential direction of the concentric tubular element (2, 3, 4) and in particular formed by pull wires which are preferably arranged within a low friction channel (54, 55, 56) extending within the concentric tubular element (2, 3, 4).

9. The system (101) according to one of the preceding claims, wherein the at least one bending section (21, 31, 41), in particular all bending sections (21, 31, 41), extends from an intermediate section between the proximal end (22, 32, 42) and a distal end (23, 33, 43) of the concentric tubular element (2, 3, 4) to the distal end (23, 33, 43) of the concentric tubular element (2, 3, 4).

10. The system (101) according to one of the preceding claims, wherein the innermost concentric tubular element (3, 4) concentrically surrounds at least one lumen (14) of the robotic endoluminal device (1) for insertion of surgical instruments (15), in particular for insertion of a guidewire.

11. The system (101) according to one of the preceding claims, wherein a maximum radial dimension of the concentric tubular elements (2, 3, 4) is essentially constant along the longitudinal direction (L) of the concentric tubular elements (2, 3, 4).

12. The system (101) according to one of the preceding claims, wherein the bending section (21, 31, 41) comprises a plurality of at least partially circumferentially extending notches (9) which are at least partially spaced apart in the longitudinal direction (L).

13. The system (101) according to claim 12, wherein at least two notches (9), in particular at least three notches (9), extend in succession along the circumferential direction of the bending section (21, 31, 41) and are circumferentially separated from each other by a material linker (91) of the bending section (21, 31, 41).

14. The system (101) according to claim 13, wherein circumferential positions of material linkers (91) of longitudinally adjacent notches (9) are offset from each other, in particular offset from each other such that the circumferential center of the notches (9) is aligned with a longitudinally adjacent material linker (91) in the longitudinal direction.

15. The system (101) according to one of the claims 13 or 14, wherein the material linkers (91) are arranged with respect to each other at an angle relative to a central axis of the bending section (21, 31, 41) of 60°, 90°, 120°, or 180°.

16. The system (101) according to one of the preceding claims, wherein the robotic endoluminal device (1) comprises a mounting section for mounting an implant, in particular a prosthetic heart valve prosthesis or a gastrointestinal end effector, in particular a suturing device.

17. A robotic endoluminal device (1) for advancement in a body duct of a patient, in particular for advancement from the femoral artery to the ascending aorta of a patient or within the gastrointestinal tract of a patient, the robotic endoluminal device (1) comprising
an outer concentric tubular element (2) and at least one inner concentric tubular element (3, 4) which are nested within each other and are independently movable in a longitudinal direction (L) of the robotic endoluminal device (1) to longitudinal positions (P1, P2, P3), wherein
an outer bending section (21) extends at least partially along the outer concentric tubular element (2) and at least one inner bending section (31, 41) extends at least partially along the at least one inner concentric tubular element (3, 4) in the longitudinal direction (L), and
at least one deflecting means (51, 52, 53) per bending section (21, 31, 41) for deflecting the respective bending section (21, 31, 41) to a curve (C1, C2, C3) and freezing the bending section (21, 31, 41), the deflecting means (51, 52, 53) extending from a proximal end (102) of the robotic endoluminal device (1) to the bending section (21, 31, 41) and the robotic endoluminal device (1) is adapted to be operatively controlled by a controller (10) for sequentially:
- Moving the outer concentric tubular element (2) to a first longitudinal position (P1) and deflecting the outer bending section (21) to a first curve (C1),
- Freezing the outer bending section (21) by the respective deflecting means (51, 52, 53),
- Moving the at least one inner concentric tubular element (3, 4) to at least a second longitudinal position (P2, P3) such that the inner bending section (31, 41) protrudes partially or completely from the outer bending section (21) and deflecting the at least one inner bending section (31, 41) to a second curve (C2, C3), and
- Freezing the at least one inner bending section (31, 41) by the respective deflecting means (51, 52, 53).

18. The robotic endoluminal device (1) according to claim 17, wherein the robotic endoluminal device (1) has a first inner concentric tubular element (3) and at least a second inner concentric tubular element (4), wherein a second inner bending section (41) extends at least partially along the second inner concentric tubular element (4) in the longitudinal direction (L) and
the second inner concentric tubular element (4) is nested within the first inner concentric tubular element (3), and the robotic endoluminal device (1) is adapted to be operatively controlled by a controller (10) for:
- Moving the second inner concentric tubular element (4) to at least a third longitudinal position (P3) such that the inner bending section (41) protrudes partially or completely from the first inner bending section (31) and for deflecting the second inner bending section (41) to a third curve (C3), and
- Freezing the second inner bending section (41) by the respective deflecting means (51, 52, 53).
